# EUROPEAN PATENT APPLICATION

(11) **EP 1 767 618 A1**
(43) Date of publication of application: **28.03.2007**
(21) Application number: 05743839.2
(22) Date of filing: 26.05.2005
(51) Int. Cl.: C12N 5/06, C12N 5/08

(54) **INDUCTION OF INSULIN SECRETING CELL**

(30) Priority: 03.06.2004 JP 2004165367
(71) Applicant: Kyoto University, Kyoto-shi, Kyoto 606-8501 (JP); JCR PHARMACEUTICALS Co., LTD., Ashiya-shi Hyogo-ken 659-0021 (JP)
(72) Inventor: SEINO, Susumu, 6590093 (JP); MINAMI, Kohtaro, 1208-5, Iwakuranagatani-cho, Kyoto-shi Kyoto 6060026 (JP); OKUNO, Masaaki, Kyoto-shi Kyoto 6068316 (JP); MIYAWAKI, Kazumasa, 831 Fujinomori godosyukusya, Kyoto-shi Kyoto 6120031 (JP)
(74) Representative: Behnisch, Werner
(86) International application number: PCT/JP2005/009613
(87) International publication number: WO 2005/118781

(57) **Abstract**

Disclosed is a method for producing insulin secreting cells to be used in cell transplantation therapy of diabetes, by induction from pancreatic acinar cells in culture vessels. The method comprises culturing pancreatic acinar cells isolated from a mammal in a medium containing a growth factor, preferably epidermal growth factor, fibroblast growth factor and/or hepatocyte growth factor, to induce insulin secreting cells.

## Description

### [TECHNICAL FIELD]

The present invention relates to induction of insulin secreting cells from insulin non-secreting cells, and more specifically, to induction of insulin secreting cells from pancreatic acinar cells.

### [BACKGROUND ART]

Since diabetes is posing serious problems, both medical or social, including (1) increasing numbers of patients (in Japan, 7.4 million patients together with 8.8 million would-be future patients), (2) development of severe complications (loss of sight from retinopathy, renal failure from nephropathy, cardiac infarction from diabetic arteriosclerosis, cerebral infarction), and (3) increasing cost of diabetes-related medical care, a pressing need exists for some fundamental solution to the problems. Prevention of the development of complications, in particular, is essential to the quality of life (QOL) of the patients.

The endstage picture of diabetes is a condition which is characterized by lack of insulin (insulin-dependency) as a result of destruction and/or malfunctioning of pancreatic β-cells. Since the discovery of insulin, its administration has been the one and only actual treatment of insulin-dependent diabetic patients. However, in quite a contrast to normal healthy persons in whom the pancreatic β-cells work in a real-time manner to detect blood sugar levels and secrete just a needed amount of insulin, it is impossible, by insulin administration, to control blood sugar levels in a real time manner so that it may fall within a normal range. Far from it, it is difficult by that therapy even to continuously supply a predetermined amount of insulin to the circulating blood of the patient. Thus, insulin administration cannot be a fundamental treatment, and prevention of diabetic complications by it, therefore, is difficult. Compared with this, either transplantation of the pancreas or pancreatic islets transplantation has a potential to provide a fundamental treatment. These methods, however, are not very practical due to the lack of donors. Transplantation into a patient of insulin secreting cells derived from stem cells by induced differentiation is thought to make available a real time control of blood sugar levels, thus providing a very promising therapy that could prevent the development of complications. While presence of stem cells is suggested also in the pancreas of adults, and they are thought to participate in regeneration of the endocrine cells (Bonner-Weir and Sharma, J. Pathol., 197:519(2002)), possibility also has been discussed that mature pancreatic duct cells or pancreatic acinar cells nay be transformed into endocrine cells by transdifferentiation (Bouwens, Microsc. Res. Tech., 43:332(1998)). Thus, the real picture has not yet become available.

In pancreatic islets transplantation, which has already been started as a fundamental treatment of diabetes, two to three donor pancreases, on an average, are required at a time (Ryan et al., Diabetes 51:2148(2002)). In performing pancreatic islets transplantation, pancreatic exocrine tissues are discarded as being of no use. Pancreatic islets, which are used for transplantation, make up only 2-3 % of the whole pancreas, and most of the remaining part consists of exocrine tissues including pancreatic acinar cells. If insulin secreting cells could be made from pancreatic acinar cells as a starting material, which occur in such a great amount and are available whenever pancreatic islets transplantation is performed, there would be a possibility that a sufficient amount of cells for transplantation be obtained from a single donor pancreas. While pancreatic acinar cells are cells which are engaged in production and secretion of digestive enzymes, they are cells embryologically sharing the same origin with pancreatic endocrine cells and difference between them is not clear until a certain stage along the differentiation (see Non-patent document 1). There have been some reports on plasticity of pancreatic acinar cells (see Non-patent document 2), but, in vitro, there are only some report that those cells undergo transformation into pancreatic duct cell-like cells (see Non-patent documents 3-5), and insulin secreting cells, thus, have not been created from them. Therefore, it is necessary to examine whether insulin secreting cells needed for transplantation therapy could be induced from pancreatic acinar cells in vitro, and, if it is found possible, to find out the method for performing it.
[Non-patent Document 1] Slack, Development, 121:1569(1995)
[Non-patent Document 2] Bouwens, Microsc. Res. Tech., 43:332(1998)
[Non-patent Document 3] Rooman et al., Diabetologia, 43:907(2000)
[Non-patent Document 4] Gmyr et al., Diabetes, 49:1671(2000)
[Non-patent Document 5] Wagner et al., Gastroenterology, 122:1898(2002)

### [PROBLEM TO BE SOLVED BY THE INVENTION]

Against the above-mentioned background, the objective of the present invention it to produce insulin secreting cells, which is to be used in cell transplantation therapy of diabetes, by induction from pancreatic acinar cells in culture vessels.

### [MEANS TO SOLVE THE PROBLEM]

The present inventors found that insulin secreting cells can be induced from pancreatic acinar cells by culturing the latter in the presence of a growth factor such as epidermal growth factor, and also that insulin secreting cells can be likewise induced even from pancreatic acinar cells of diabetic animals. The present invention has been made on the basis of these findings.

Thus, the present invention provides what follows:
1. A method for producing insulin secreting cells from non-insulin producing cells of a mammal, comprising culturing pancreatic acinar cells isolated from a mammal in a medium containing a growth factor to induce insulin secreting cells.
2. The method for production as defined in 1 above comprising collecting induced insulin secreting cells included in the cultured cells.
3. The method for production as defined in 2 above, wherein collection of insulin secreting cells is carried out by collecting colonies containing induced insulin secreting cells.
4. The method for production as defined in one of 1 to 3 above, wherein the growth factor is epidermal growth factor (EGF), fibroblast growth factor 10 (FGF10) and/or hepatocyte growth factor (HGF).
5. The method for production as defined in one of 1 to 4 above, wherein the culture is performed in the presence of a growth factor at a concentration of at least 10 ng/ml.
6. The method for production as defined in one of 1 to 5 above, wherein the culture is performed at least until a colony is formed which is made of a cell aggregate consisting of smaller cells than surrounding cells.
7. The method for production as defined in one of 1 to 6 above, wherein the culture is performed under the condition where the concentration of fetal bovine serum is 0-2 %.
8. An insulin secreting cell produced by the method as defined in one of 1 to 7 above.

### [EFFECT OF THE INVENTION]

The present invention enables to obtain insulin secreting cells, which are for transplantation to patients for the treatment of diabetes, from pancreatic acinar cells available in quite a greater amount than pancreatic β-cells.

### [BRIEF DESCRIPTION OF DRAWINGS]

[FIG. 1] Fig. 1A is a photomicrograph of acinar cells at the initial stage of culture, and Fig. 1B a photomicrograph of a cluster of the acinar cells 7 days after culture.
[FIG. 2] Fig. 2, A-C, upper row, indicate fluorescent photomicrographs of insulin positive cells, and A-C, lower row, which are fluorescent photomicrographs of the same cell clusters shown in A-C in the upper row, show PCNA-, glucagon- and insulin-positive cells, respectively.
[FIG. 3] Fig. 3 is a graph illustrating the proportion (%) of insulin-positive colonies on days 3 and 6 of culture of the pancreatic acinar cells.
[FIG. 4] Fig. 4 shows fluorescent photomicrographs indicating correlation between the concentration of fetal bovine serum and generation of insulin-positive cells.
[FIG. 5] Fig. 5 shows photomicrographs (left) and fluorescent photomicrographs (right) of the cell clusters containing insulin-positive cells induced in uncoated (A), laminin-coated (B) and poly-D-lysine-coated culture dishes, respectively.
[FIG. 6] Fig. 6 shows photomicrographs of pancreatic islets of a normal mouse and a streptozotocin-treated mouse.
[FIG. 7] Fig. 7 shows fluorescent photomicrographs indicating a colony (left: nuclear staining) and insulin-positive cells in the colony (right), induced from pancreatic acinar cells of a streptozotocin-treated mouse.
[FIG. 8] Fig. 8 shows graphs illustrating the effect of growth factors on acceleration of cell growth (A) and on induction of insulin-positive cells (B), of growth factors.
[FIG. 9] Fig. 9 shows photomicrographs indicating the correlation between EGF concentration and the induction effect.
[FIG. 10] Fig. 10 shows a graph illustrating the release of insulin into the medium from induced insulin-positive cells.
[FIG. 11] Fig. 11 shows a photomicrograph (A) of a pancreatic acinar cell aggregate on day 7 of culture on Matrigel (TM) matrix and a fluorescent photomicrograph (B) of insulin-positive cells in the same cell aggregate.
[FIG. 12] Fig. 12 shows a graph illustrating comparison of insulin content in cells between those cultured in non-treated dishes and Matrigel (TM)-coated ones.
[FIG. 13] Fig. 13 shows fluorescent photomicrographs of cell clusters induced from pig pancreatic acinar cells (A) and insulin-positive cells (B).

### [BEST MODE FOR CARRYING OUT THE INVENTION]

In the present invention, a "mammal" includes a human, a pig and a mouse. As described bellow, it has been confirmed in the present invention that insulin secreting cells are induced not only from acinar cells of a mouse but also those of a pig under the same conditions.

In the present invention, an "insulin secreting cell" means a cell that has the ability of producing insulin and releases it into the surrounding medium.
In the present invention, an "insulin positive cell" means a cell which is detectable as containing insulin by any detection means as desired. An insulin positive cell is equivalent to an insulin secreting cell, since harvested insulin positive cells have been confirmed to secrete insulin into the medium, as described later.

In the present invention, culture of pancreatic acinar cells is conducted in, preferably, a liquid medium. Insulin secreting cells thus induced are contained in colonies consisting of cells which are smaller than surrounding cells, and the cells which are aimed at may be harvested e.g., by pipetting such colonies under a microscope.

Examples of growth factors that are to be incorporated in the growth medium for pancreatic acinar cells include epidermal growth factor (EGF) and fibroblast growth factor 10 (FGF10). Of these, epidermal growth factor is particularly preferred. The concentration of epidermal growth factor is preferably not less than 10 ng/ml, and may be, e.g., 10, 15 or 20 ng/ml, although higher concentrations than these, such as 200 ng/ml, are also allowed.

In the present invention, the culture may be conducted either in the presence (e.g., 10 %) or absence of fetal bovine serum. Though presence of fetal bovine serum is preferred for the purpose of maintenance of, and acceleration of the growth of, mammalian cells, it accelerates not only the growth of the insulin secreting cells, the very aimed cells induced, but also the other cells. Therefore, it is preferable to terminate the total growth of the cells at a level that will not hinder the collection of the aimed cells after the completion of culture. A concentration may be determined as desired in accordance with the density of the cells inoculated in the culture vessel and the number of days for which culture is to be continued. Generally, fetal bovine serum at the concentration of not more than 2 % would not hinder easiness of handling, but it is also allowed not to include any fetal bovine serum.

Culture of pancreatic acinar cells may be conducted at a temperature at which culture of mammalian cells are generally performed, e.g., 37 °C. At this temperature, insulin secreting cells are induced in, e.g., 5-8 days of culture.

Through their injection into the portal vain, for example, it is possible to let the insulin secreting cells obtained according to the present invention take root in the liver and secrete insulin there. In this case, it will be of particular advantage if insulin secreting cells used are those induced from the pancreatic acinar cells taken out from the patient's own pancreas, for they will cause no immune reactions.

### [EXAMPLE 1]

### Method for Induction of Insulin-positive Cells

### <Isolation of Pancreatic Acinar cells>

Eight-week old male mice (C57Bl/6Cr Slc)(Japan SLC, Inc.: Hamamatsu, Japan) were anesthetized with 50 mg/kg of Nembtal (Dainippon Pharma Co., Ltd.: Osaka, Japan) and subjected to laparotomy. The choledoch duct was ligated near the position where it opens to the duodenum. Using a 27G winged needle (Terumo Corporation: Tokyo, Japan), 2-3 ml of Hank's buffer containing 0.05 % collagenase P (Roche: Basel, Switzerland) and 10 % fetal bovine serum was infused from the liver side. The pancreas, now swollen, was extracted and digested in 5 ml of Hank's buffer per pancreas for 18 minutes at 37 °C. The digested pancreas then was made to pass through a 14G indwelling needle in both directions twice to disperse the pancreatic cells, which was washed well with Hank's buffer containing 0.1 % bovine serum albumin (Sigma: Saint Louis, MO, USA). The pancreatic cells thus obtained were suspended in a mixed solution (solution A) consisting of 8.3 % Ficoll 400 (Amersham Bioscience: Uppsala, Sweden) and 16.7 % Conray 400 (Daiichi Pharmaceutical, Tokyo, Japan), and after a 87.5 %-diluted solution of solution A and then a 50 %-diluted solution of solution A were placed on it in the order, centrifuged (2,000 rpm, 10 min). Pancreatic acinar cells condensed in the spun down, lowest fraction were collected, and to them was added a sterilized 0.1 mg/ml solution of dithizone (1,5-diphenylthiocarbazone) (Nacalai Tesque, Inc.: Kyoto, Japan) and stained for 15 minutes at 37 °C in order to remove contaminating mature β-cells. Mature β-cells are stained red with dithizone, which is a chelating agent for zinc, for high concentration of insulin occurs in mature β-cells in the form of a hexamer via zinc (Latif et al., Transplantation, 45:827(1988)). By removing red-stained mature β-cells under a stereomicroscope, a fraction containing highly concentrated pancreatic acinar cells were obtained.

### <Pre-culture of Pancreatic Acinar Cells>

Isolated pancreatic acinar cells were suspended in a nutrient medium (e.g., RPMI-1640) containing 10 % fetal bovine serum (Sigma) and inoculated in 90-mm culture dishes (Asahi Techno Glass: Funabashi, Japan), so that the pancreatic acinar cells from one animal are assigned to one dish, and cultured for 6 hours in an incubator which was set at 37 °C and 5 % CO₂. While fibroblasts, being of adhesive property, adhered during this period, pancreatic acinar cell would not. By recovering cells which were floating, pancreatic acinar cells were obtained.

### <Main Culture of Pancreatic Acinar Cells>

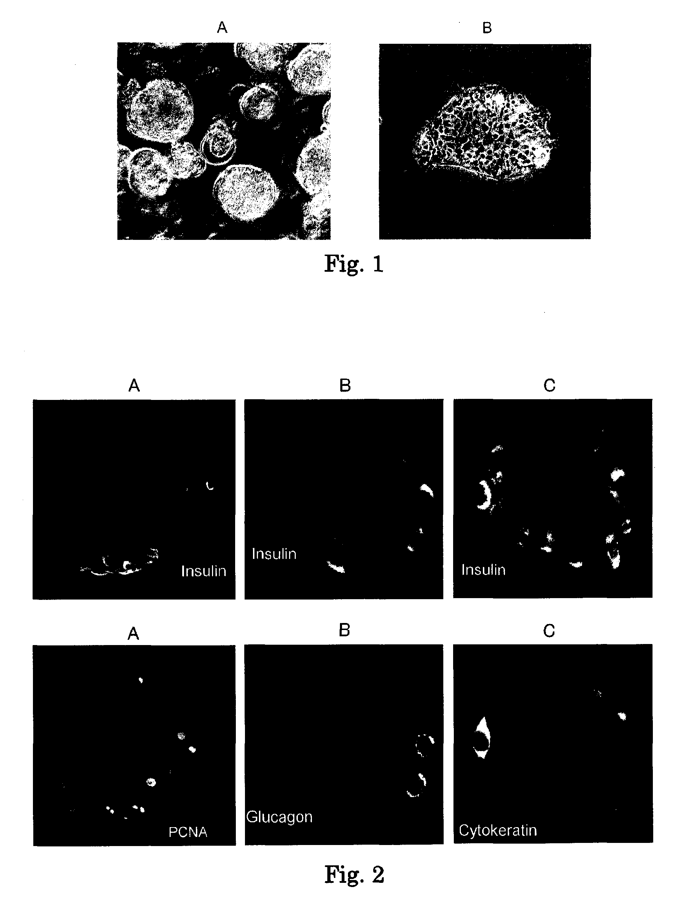

The recovered cells were suspended in a nutrition medium (e.g., RPMI-1640) containing 0.5 % fetal bovine serum (Sigma) and 20 ng/ml epidermal growth factor (EGF) (R&D Systems: Minneapolis, MN, USA), and inoculated in cell culture dishes which were less adhesive for cells (Nalge Nunc: Rochester, NY, USA) and cultured in an incubator set at 37 °C, 5 % CO₂. The pancreatic acinar cells, at early stages of culture, formed cell clusters having smooth boundaries consisting of congregated cells and, and kept themselves floating (Fig. 1A), and, at around day 3, adhere to the bottom of the dish and started to extend on it. After about 7 days, they formed relatively small cell aggregates (colonies) consisting of several dozen to several hundred cells (Fig. 1B). The cell density upon inoculation, which ranged from 1- to 10-fold dilution of that in pre-culture, had no influence on the form of the colonies observed.

### <Immunostaining>

The cells which adhered to the cell culture dish were washed with phosphate buffered physiological saline (PBS), and fixed with 4 % paraformaldehyde (Wako Pure Chemical Industries: Osaka, Japan) for 20 minutes at room temperature. The cells then were washed with PBS three times, 5 minutes each, and blocked for 20 minutes at room temperature with PBS containing 10 % normal goat serum (Chemicon: Temecula, CA, USA) and 0.2 % Tween-20 (ICN Biomedicals: Aurora, Ohio, USA). Primary antibodies diluted with PBS containing 1 % normal goat serum were added and let react for 2 hours at room temperature. The primary antibodies employed were a guinea pig anti-insulin antibody (2-fold dilution) (Zymed: South San Francisco, CA, USA), a mouse anti-glucagon antibody (1,000 fold dilution) (Sigma), a rabbit anti-PCNA (proliferating cell nuclear antigen) antibody (200-fold dilution) (Santa Cruz Biotechnology, Inc.: Santa Cruz, CA, USA), or a mouse anti-cytokeratin antibody (50-fold dilution) (Dako: Carpinteria, CA, USA). After washing three times with PBS, 5 minutes each, secondary antibodies diluted with PBS containing 1 % normal goat serum were added and let react for 1 hour at room temperature. The secondary antibodies employed were Alexa Fluor 488 goat anti-guinea pig IgG antibody (200-fold dilution) (Molecular Probes, Eugene, OR, USA), Alexa Fluor 546 goat anti-mouse IgG antibody (200-fold dilution) (Molecular Probes), or Alexa Fluor 546 goat anti-rabbit IgG antibody (200-fold dilution) (Molecular Probes). After washing three times with PBS, 5 minutes each, nuclear staining with 2.5 µg/ml DAPI (4', 6'-diamidino-2-phenyl-indol) (DOJINDO LABORATORIES: Kumamoto, Japan) was performed. Observation was made through a fluorescence microscope (Olympus: Tokyo, Japan) and the images were recorded with a cooled digital CCD camera (Hamamatsu Photonics K.K.: Hamamatsu, Japan).

In the colonies after one week of culture, cells recognized by anti-insulin antibody (insulin-positive cells) were noted (Fig. 2, upper row, A-C). Part of the insulin-positive cells were proved to be growing cells, since they were PCNA positive (Fig. 2, lower row, A). Part of the insulin-positive cells were also positive simultaneously to the other hormones, i.e., glucagon (Fig. 2, lower row B) and to cytokeratin, a marker of pancreatic duct cells (Fig. lower row, C). Since β-cells, at certain stage of the development, are insulin/glucagon-positive and many types of cells including β-cells differentiate from a duct-like structure (Slack, Development, 121:1569(1995)), the insulin-positive cells observed above are thought to be cells which are on their way of differentiation from pancreatic acinar cells, and not contaminating β-cells.

### [EXAMPLE 2]

### <Study of Length of Culture >

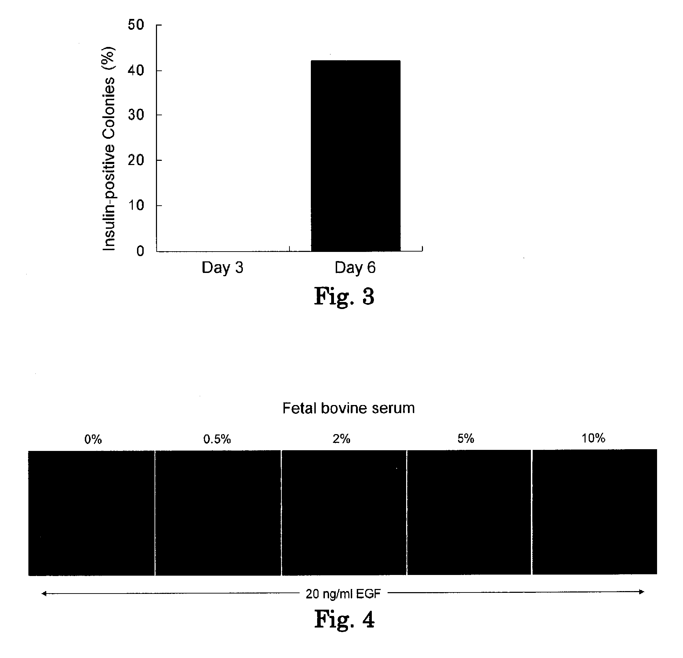

**T**o find out the optimal length of culture, pancreatic acinar cells of the mouse isolated by the method described in Example 1 (i.e., floating cells recovered from the pre-culture) were subjected to the main culture in the same manner as described in Example 1. Immunostaining of insulin was performed on days 3 and 6 of culture, and the proportion of the colonies containing insulin-positive cell was determined. Insulin-positive cells were hardly observed on day 3 of the main culture, but on day 6 of the culture, about 40 % of the colonies were found containing insulin-positive cells (Fig. 3). It is thought that the greatest number of insulin-positive cells can be obtained around one week after the start of culture.

### [EXAMPLE 3]

Mouse pancreatic acinar cells isolated by the method described in Example 1 were subjected to the main-culture in the same manner as described in the example, except that the concentration of fetal bovine serum was adjusted to 0 %, 0.5 %, 2 %, 5 % or 10 %, and examined for generation of insulin-positive cells. The medium which was used for the 0 % fetal bovine serum group contained 1 % albumin (Sigma). Insulin-positive cells were found induced at any one of the serum concentrations tested, (Fig. 4). When the concentration of fetal bovine serum was 5 or 10 %, relatively enhanced growth of other cells than insulin-positive cells were also noted. Therefore, from the view point of easier collection of insulin-positive cells, the concentration of fetal bovine serum should be suppressed. For example, it may be not more than 2 %. It is also allowed not to include any of the serum.

### [EXAMPLE 4]

### <Study of Coated Dishes>

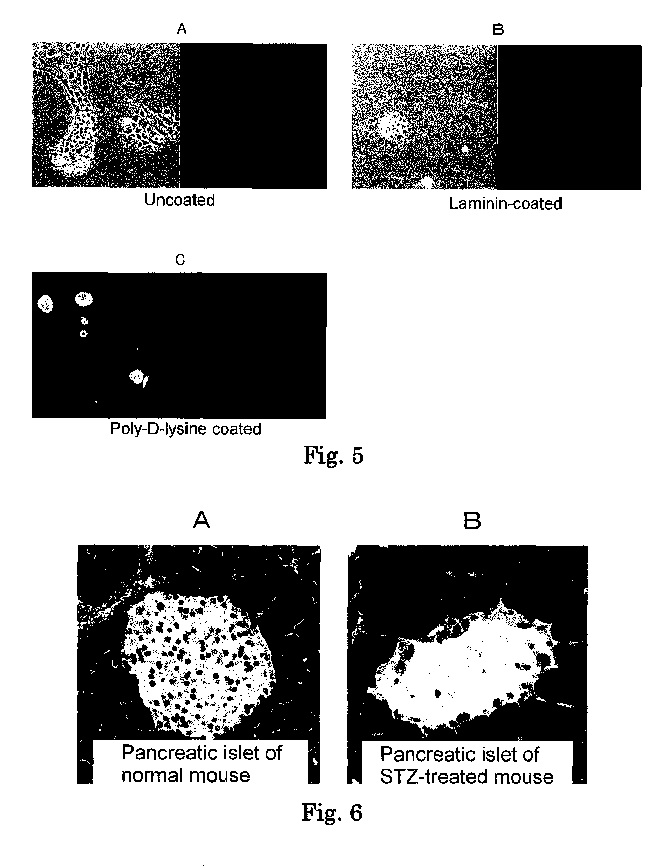

Under the culture conditions described in Example 1, pancreatic acinar cells were inoculated and cultured in each of; (A) uncoated cell culture dishes, (B) laminin-coated cell culture dishes (Becton, Dickinson), and (C) poly-D-lysine-coated cell culture dishes (Becton Dickinson). Induction of insulin positive cells was observed in all the dishes (Fig. 5, A-C: photomicrographs of colonies (left) and fluorescent photomicrographs of insulin-positive cells contained in them (right), respectively).

### [EXAMPLE 5]

### <Induction of Insulin-positive Cells from Pancreatic Acinar Cells of Type-I Diabetic (β-cells-destroyed) Mouse>

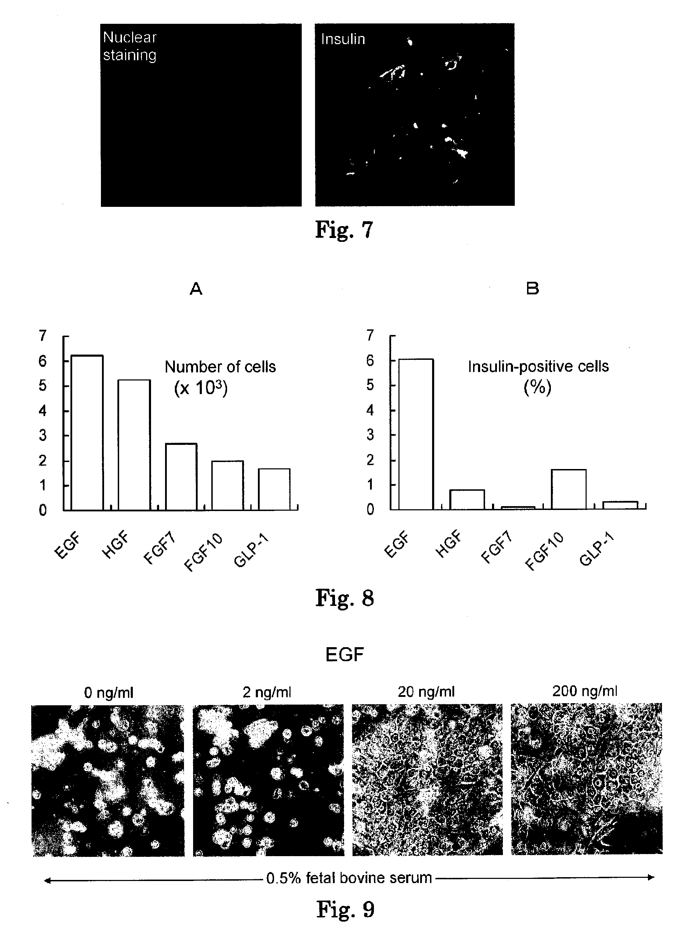

Streptozotocin (STZ) (Sigma) was dissolved in citrate buffer (pH 4.5) and 200 mg/kg of it was injected into the abdominal cavity of mice that had been fasted overnight. Two days later, the mice were confirmed to have blood sugar levels over 400 mg/dl, and then used for experiment. Destruction of β-cells was confirmed by hematoxylin-eosin staining of a pancreatic section (Fig. 6A, B). Isolation and culture of pancreatic acinar cells were carried out as described in Example 1. Detection by immunostaining showed induction of insulin-positive cells with a ratio comparable to that observed with pancreatic acinar cells from normal mice (Fig. 7). This indicates that the insulin-positive cells obtained by the method had differentiated from other cells than β-cells (therefore, from pancreatic acinar cells) and also that they can be produced from Type-I diabetic patients' pancreases in which the β-cells have been destroyed. The results suggest the possibility of treating diabetes by autotransplantation.

### [EXAMPLE 6]

### <Effect of Growth Factors on the Efficiency of Insulin-positive Cell Induction>

Studies were performed to examine the effect of growth factors on insulin-positive cell induction, using EGF (R&D Systems), hepatocyte growth factor (HGF) (R&D Systems), fibroblast growth factor 7 (FGF7) (R&D Systems), fibroblast growth factor 10 (FGF 10) (R&D Systems), and glucagon-like peptide-1 (GLP-1) (Peptide Institute, Inc., Osaka, Japan). In accordance with the method described in Example 1, except that one of those growth factors was further added at its standard concentration for use, i.e., 20 ng/ml for EGF, 10 ng/ml for HGF, FGF7 and FGF10 and 30 ng/ml for GLP-1, main culture of pancreatic acinar cells was performed. On day 7 of culture, the total number of cells was counted and the proportion of insulin-positive cells was determined. For total cell count (growth of the cells), the most effective growth factor was EGF, and this was followed by HGF (Fig. 8A). For the proportion of insulin-positive cells, the most effective growth factor was EGF, with remarkable difference from other growth factors, and this was follwed by FGF10 and HGF followed (Fig. 8B).

### [EXAMPLE 7]

### <Examination of Correlation between EGF Concentration and Induction Effect>

In accordance with the method described in Example 1, except that the concentration of fetal bovine serum was fixed at 0.5 % and EGF was used as the growth factor at a concentration of 0, 2, 20 or 200 ng/ml, main culture of pancreatic acinar cells were performed to assess the effect of EGF on induction of aimed cells (cells forming colonies containing insulin-positive cells as shown in Fig 1B). As a result, remarkable effect was observed at EGF concentrations of 20 and 200 ng/ml (Fig. 9).

### [EXAMPLE 8]

### <Insulin Secreting Reaction>

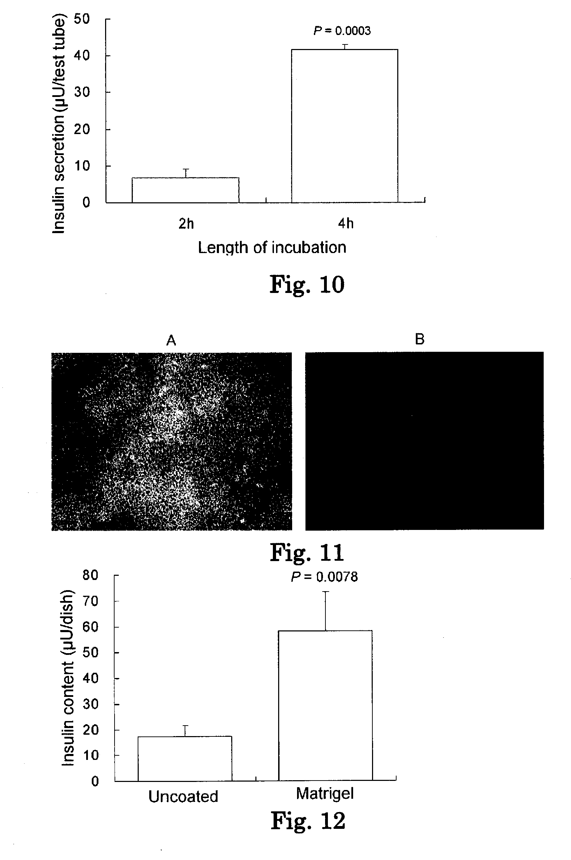

Insulin secreting reaction from the insulin-positive cells induced as above was examined. Recovered cells were suspended in 500 µl of Krebs buffer containing 0.1 % bovine serum albumin (approx. 2.5 × 10⁵ cells per test tube), and were stimulated with glucose at a concentration of 25 mM for 4 hours at 37 °C. Secreted insulin was measured by ELISA using a kit purchased from Shibayagi K.K.. Significant increase in insulin secretion was noted from 2 to 4 hours (Fig. 10).

### [EXAMPLE 9]

### <Improvement in Efficiency of Induced Differentiation into Insulin-positive Cells by Culture on Matrigel (TM)>

For the cells to grow and differentiate, an extracellular environment providing scaffolds to them is important. In the body, basement membranes are present containing a variety of extracellular matrixes, and they also serve as a reservoir of, e.g., growth factors. Use of Matrigel (TM) matrix is known as a method for reproducing such an environment in vitro and reported to accelerate differentiation of various cells (Kleinman et al., Biochemistry, 25:312(1986), Hadley et al., J. Cell. Biol., 101:1511(1985), Kubota et al., J. Cell. Biol., 107:1589(1988), McGuire and Orkin, Biotechniques, 5:456(1987)). Matrigel (TM) matrix, whose chief components are laminin, collagen IV, heparan sulfate, proteoglycan and entactin, also contains insulin-like growth factor (IGF-I) in relatively a high amount. Seeking to improve the efficiency of differentiation into insulin-positive cells, culture was tried on a Matrigel (TM) matrix.

One to four days after the start of the main culture done as described in Example 1, floating clusters of pancreatic acinar cells were collected and re-inoculated into thin layer Matrigel (TM)-coated dishes (Becton, Dickinson). Since the following day, the cell clusters adhered to the dish and started to expand, exhibiting vigorous growth. Inside of colonies that grow big were formed a number of agglomerates of smaller cells clearly distinguished from surrounding cells (Fig. 11A). Immunostaining after one- to seven-day culture on Matrigel (TM) revealed that insulin-positive cells were localized in the agglomerates of those smaller cells, and insulin-positive cells occurred at higher ratio in the agglomerates (Fig. 11 B).

In order to compare, between Matrigel (TM)-coated dishes and untreated ones, the efficiency of induction of differentiation into insulin-positive cells from pancreatic acinar cells, insulin content of the cells was measured after completion of the culture. Briefly, acidic ethanol (prepared by addition, 15/1000 in volume, of conc. hydrochloric acid to 75 % ethanol) was added to the post-culture cells, and the culture was allowed to stand for 24 hours at 4 °C, and the supernatant was collected, diluted and measured for insulin content by ELISA using a kit purchased from Shibayagi K. K.. Thus, it was found that culture on Matrigel (TM) boosted insulin content not lower than 3 times that of culture in untreated dishes (Fig. 12).

### [EXAMPLE 10]

### <Induction of Insulin-positive Cells from Pig Pancreatic Acinar Cells>

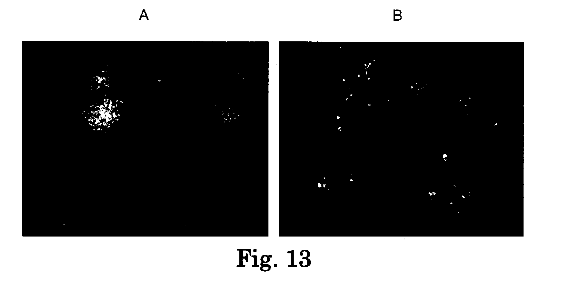

Pancreatic acinar cells of a mature pig were extracted and cultured by the method described in Example 1. This confirmed that induction of insulin-positive cells occurred just as observed in the case of mouse (Fig. 13A, nuclear staining, Fig. 13B, insulin-positive cells).

### [INDUSTRIAL APPLICABILITY]

Instead of pancreatic β-cells that are available only in a very small amount, the present invention enables to produce insulin secreting cells from pancreatic acinar cells, which can be obtained in relatively a greater amount,

## Claims

1. A method for producing insulin secreting cells from non-insulin producing cells of a mammal, comprising culturing pancreatic acinar cells isolated from a mammal in a medium containing a growth factor to induce insulin secreting cells.

2. The method for production of claim 1 comprising collecting induced insulin secreting cells included in the cultured cells.

3. The method for production of claim 2, wherein collection of insulin secreting cells is carried out by collecting colonies containing induced insulin secreting cells.

4. The method for production of one of claims 1 to 3, wherein the growth factor is epidermal growth factor (EGF), fibroblast growth factor 10 (FGF10) and/or hepatocyte growth factor (HGF).

5. The method for production of one of claims 1 to 4, wherein the culture is performed in the presence of a growth factor at a concentration of at least 10 ng/ml.

6. The method for production of one of claims 1 to 5, wherein the culture is performed at least until a colony is formed which is made of a cell aggregate consisting of smaller cells than surrounding cells.

7. The method for production of one of claims 1 to 6, wherein the culture is performed under the condition where the concentration of fetal bovine serum is 0-2 %.

8. An insulin secreting cell produced by the method of one of claims 1 to 7.
